Europäisches Patentamt

(19) European Patent Office

Office européen des brevets

(11) Publication number: **0 178 464**
**A2**

(12) **EUROPEAN PATENT APPLICATION**

(21) Application number: **85111606.1**

(22) Date of filing: **13.09.85**

(51) Int. Cl.⁴: **A 61 B 17/22**
**A 61 B 17/36**

(30) Priority: **17.09.84 US 650889**

(43) Date of publication of application:
**23.04.86 Bulletin 86/17**

(84) Designated Contracting States:
**DE FR GB IT**

(71) Applicant: **Xintec Corporation**
**900 Alice Street**
**Oakland California 94607(US)**

(72) Inventor: **Lee, Garrett**
**223 Scenic Avenue**
**Piedmont California 94611(US)**

(74) Representative: **UEXKÜLL & STOLBERG Patentanwälte**
**Beselerstrasse 4**
**D-2000 Hamburg 52(DE)**

(54) Laser revascularization device and method of operation therefor.

㋗ A device for luminal recanalization of atherosclerotic blood vessels includes a guide sleeve adapted to be introduced into the blood vessel and advanced to the atherosclerotic plaque. The sleeve includes a fluid passage to conduct fluid to the distal end to inflate an exterior-mounted balloon to immobilize the sleeve and block blood flow in the vessel. A fiberoptic core is adapted to be advanced through the guide sleeve, with spacing therebetween to permit the flow of cooling fluid to the distal end of the sleeve. The distal end of the fiberoptic core is provided with a heat conductive cap which is adapted to be selectively heated by laser energy delivered through the core. The cap is advanced beyond the distal end of the sleeve to contact the plaque, and the laser energy virtually instantaneously heats the cap to cause thermal destruction of the plaque. The core may be removed to flush the plaque debris from the vessel, and to introduce an endoscopic fiberoptic core to visualize the results. In tortuous vessels, a preformed guide wire may first be inserted into the vessel, the guide sleeve advanced about the guide wire into the vessel, and the wire removed and replaced by the fiberoptic core. In a related embodiment the guide sleeve is provided with parallel channels to receive the fiberoptic core and the guide wire simultaneously, so that the sleeve may be advanced through a vessel along a guide wire with the cautery cap extended and heated reiteratively as required to remove plaque as it is encountered.

FIG _ 1

EP 0 178 464 A2

## LASER REVASCULARIZATION DEVICE
## AND METHOD OF OPERATION THEREFOR

### BACKGROUND OF THE INVENTION

The first medical application of the concentrated, directed energy available from coherent laser light was retinal photocoagulation. Subsequently, the ability to precisely control certain characteristics of the light beam resulted in laser surgical tools which were used to excise or destroy cancerous tissue, hemangiomas, and other cutaneous lesions. With the advent of optical fibers to propagate the laser energy into internal body cavities, light could be used to visualize bleeding gastrointestinal ulcers and transmitted laser beams could be used to photocoagulate the lesions. Initial use of laser energy for the dissolution of coronary atherosclerotic disease was developed in part by the present inventors.

Almost all preclinical investigations utilized one of three laser output devices: argon-ion, neodymium-yttrium-aluminum garnet (Nd:YAG, or simply YAG), and carbon dioxide. The energy beam of the first two laser types is directed through fiberoptic catheters with the laser emission impinging directly on the atherosclerotic plaque. Due to the fact that plaques are not uniform in their content of lipid, hyaline, and calcified deposits, and that each laser produces a specific wavelength which is absorbed to greatly varying degrees by the differing deposits, these "free beam" devices are not suitable.

The use of the carbon dioxide laser is constrained by the current lack of fiberoptic material which can effectively transmit the wavelength of the $CO_2$ laser. Furthermore, this wavelength, 10.6 microns, is entirely absorbed by water in soft tissue within 100 microns of the surface impact. Rapid, localized heat production at the laser impact site causes cell water evaporation and tissue ablation, so that any effective clinical use must ensure a completely dry surgical field. This constraint renders the $CO_2$ laser unsuitable for revascularization work.

Early studies by the present inventors as well as other investigators indicated that while intraluminal laser dissolution of atherosclerotic plaque by the introduction of free laser beams of suitable wavelength resulted in the removal of plaque without enhancement of intravascular thombogenesis, the blind application of laser energy through a catheter without some means of targeting resulted in muscular wall damage in the treated vessels whose ultimate manifestations are vasular perforation aneurysm. Power intensities of one to two watts lasting three seconds, transmitted through a 400 micrometer diameter quartz fiber core attached to an argon laser, were used in one experiment. In a subsequent experiment an argon laser free beam was introduced into the right internal carotid artery of a dog and positioned under fluoroscopic guidance in the proximal left coronary artery. The delivery of 25 to 80 joules resulted in laser burns perforating the coronary artery, causing myocardial necrosis, hemorrhage, and cardiac tamponade.

These significant complications led to the development of a viewing catheter device which can simultaneously visualize and then vaporize atherosclerotic plaques. The advantage of using the visible light spectrum produced by the argon laser lies in the targeting of the laser beam impact point. While this concept appeared feasible, the optical resolution of devices currently in use did not allow an adequate level of confidence in the safety of performing laser revascularization in narrow channels where the proximity of normal vessel wall enhances the risk of damage by the laser beam.

In addition to the potential of anatomical damage to vascular structures, free beam treatment has been shown to result in lysis of red cells exposed directly to the laser beam. Since free hemoglobin in plasma was observed to rise in direct proportion to the time of laser exposure, some late manifestation of cell membrane damage in cells in the shadow of the overlying cell layer exposed to the free beam may be occurring. The long term effect of this type of intravascular damage is not known, but cannot be dismissed without further study.

In reviewing microscopic studies of free beam revascularization procedures, it can be concluded that the mechanism of action of tissue ablation can best be described as explosive evacuation. Upon impact by the incident laser beam, water and possibly other volatile components such as fats and light, proteins vaporize instantaneously within cells or other noncellular tissue. The resulting localized overpressure ruptures tissue, causing fragmentation and shattering of the tissue. In disrupted tissue, evidence of thermal damage can readily be observed as coagulation necrosis, loss of cellular integrity, and vacuolization within cellular and noncellular materials. The depth of damage is related to the total power applied to each site. Studies of calcified plaque material indicate that the explosive effect of laser energy of surrounding tissue with water or protein matrices results in transmission of the disruptive forces to the calcified deposits, which themselves appear to resist direct thermal influence by the laser free beam. Thus, calcium deposits are not invariably removed by free beam techniques.

To summarize the prior art, laser technology as applied to revascularization has not been able to provide a sufficient margin of safety using free laser beams. It has been too difficult to achieve precise control of tissue damage, resulting in an unacceptable high rate of damage to healthy surrounding tissue.

## SUMMARY OF THE PRESENT INVENTION

The present invention generally comprises a method and apparatus for luminal recanalization of atherosclerotic blood vessels using directed laser energy. However, in contrast to the prior art, the present invention does not employ a free laser beam. Rather, the invention includes a cautery cap which is virtually instantaneously heated to destroy the atherosclerotic plaque. Thus damage to surrounding healthy vascular tissue is significantly reduced or eliminated.

The present invention includes a guide sleeve adapted to be introduced into the blood vessel and advanced to the site of atherosclerotic plaque. The sleeve includes a fluid passage to conduct fluid to the distal end to inflate an exterior-mounted balloon which immobilizes the sleeve and blocks blood flow in the vessel. A fiberoptic core is adapted to be advanced through the guide sleeve, with spacing therebetween to permit the flow of cooling fluid to the distal end of the sleeve. The distal end of the fiberoptic core is provided with the heat conductive cautery cap which is adapted to be selectively heated by laser energy delivered through the core. The cap is advanced to a limited extent beyond the distal end of the sleeve to contact the plaque, and the laser energy virtually instantaneously heats the cap to cause thermal destruction of the plaque. The core may be removed to flush the plaque debris from the vessel, and to introduce an endoscopic fiberoptic core to visualize the results. In tortuous vessels, a preformed guide wire may first be inserted into the vessel, the guide sleeve advanced about the guide wire into the vessel, and the wire removed and replaced by the fiberoptic core. Alternatively, the guide sleeve may be provided with a pair of parallel bores extending therethrough to receive the guide wire and fiberoptic core simultaneously. The assembly may be advanced through the vessel, with the cap heated reiteratively to destroy plaque as it is encountered.

The cooling fluid flowing to the distal tip cools the low mass cap as soon as the laser energy ceases. Thus the thermal destruction of tissue is limited to tissue in direct contact with the cap. With the balloon centering the cap in the vessel and the limited advance of the cap, surrounding tissue is protected from damage. Furthermore, the device uses no free beam at all, so that safety precautions regarding eye protection, specular reflections of laser light, combustible materials close by, and the like are obviated.

## BRIEF DESCRIPTION OF THE DRAWINGS

FIGURE 1 is a cross-sectional side elevation of the cautery cap of the present invention.

FIGURE 2 is a cross-sectional side elevation of the junction of the cautery cap and the fiberoptic core of the present invention.

FIGURE 3 is a cutaway view of the fiberoptic core of the present invention.

FIGURE 4 is a plan view of the proximal end of the guide sleeve assembly of the present invention.

FIGURE 5 is a plan view of the revascularization instrument of the present invention.

FIGURE 6 is a cross-sectional view of the terminal hub at the distal end of the instrument of the present invention, shown with the cautery cap retracted.

FIGURE 7 is a cross-sectional view as in FIGURE 6, shown with the cautery cap extended.

FIGURE 8 is a plan view of the proximal end of the revascularization instrument of the present invention.

FIGURE 9 is a plan view of the instrument of the present invention, shown being used in the irrigation and flushing mode.

FIGURE 10 is a plan view of the instrument of the present invention, shown being used in an alternative flush and suction mode.

FIGURE 11 is a plan view of another embodiment of the present invention, shown with the cautery cap extended.

FIGURE 12 is a plan view of the present invention, shown being translated about a preformed guide wire.

FIGURE 13 is a schematic view of the instrument of the present invention, showing the guide sleeve being introduced into an occluded blood vessel.

FIGURE 14 is a schematic view as in FIGURE 13, showing the inflation of the distal exterior balloon.

FIGURE 15 is a schematic view as in FIGURE 14, showing the advancement of the cautery cap from the distal terminal hub.

FIGURE 16 is a schematic view as in FIGURE 15, showing the advancement of the cautery cap to the maximum extent allowed by the proximal stop.

FIGURE 17 is an enlarged, detailed view of the cautery cap at maximum extension from the distal terminal hub.

FIGURE 18 is a schematic view as in FIGURES 13-16, showing the introduction of fluid into the instrument.

FIGURE 19 is a perspective view of the terminal hub of the guide sleeve.

FIGURE 20 is a schematic view as in FIGURES 13-16 and 18, showing the results after the first laser power application to the cautery cap.

FIGURE 21 is a schematic view as in FIGURE 20, showing the cautery cap being retracted.

FIGURE 22 is a schematic view as in FIGURE 21, showing the advancement of the guide sleeve into the new bore formed in the atherosclerotic plaque.

FIGURE 23 is a schematic view as in FIGURE 22, showing the results of the completed revascularization procedure using the present invention.

FIGURE 24 is a schematic view as in FIGURE 23, showing the fiberoptic core being removed from the guide sleeve.

FIGURE 25 is a schematic view as in FIGURE 24, showing the flush and suction removal of the plaque debris.

FIGURE 26 is a schematic view as in FIGURE 25, showing the removal of the guide sleeve from the blood vessel.

FIGURE 27 is a schematic view showing the guide sleeve being advanced into a blood vessel about a previously inserted guide wire.

FIGURE 28 is a schematic view as in FIGURE 27, showing the injection of a contrast medium through the guide sleeve.

FIGURE 29 is a schematic view as in FIGURE 28, showing removal of the guide wire from the guide sleeve.

FIGURE 30 is perspective view of a further embodiment of the present invention which is wire guided and continuously activated.

FIGURE 31 is a cross-sectional view of the guide wire of the embodiment of FIGURE 30 disposed in a blood vessel.

FIGURE 32 is a cross-sectional view of the embodiment of FIGURE 30 as it is introduced into the blood vessel on the guide wire.

FIGURE 33 is a cross-sectional view of the embodiment of FIGURE 30, shown with the cap extended and the balloon inflated.

FIGURE 34 is a cross-sectional view as in FIGURES 30-33, showing the plaque volume removed by the device.

## DESCRIPTION OF THE PREFERRED EMBODIMENT

The present invention generally comprises a laser driven instrument which is adapted to revascularization of atherosclerotic vascular structures. A salient feature of the invention is that it achieves removal of the atherosclerotic plaque without damaging the surrounding vascular structures, and without the risks associated with the use of a laser free beam within a body cavity.

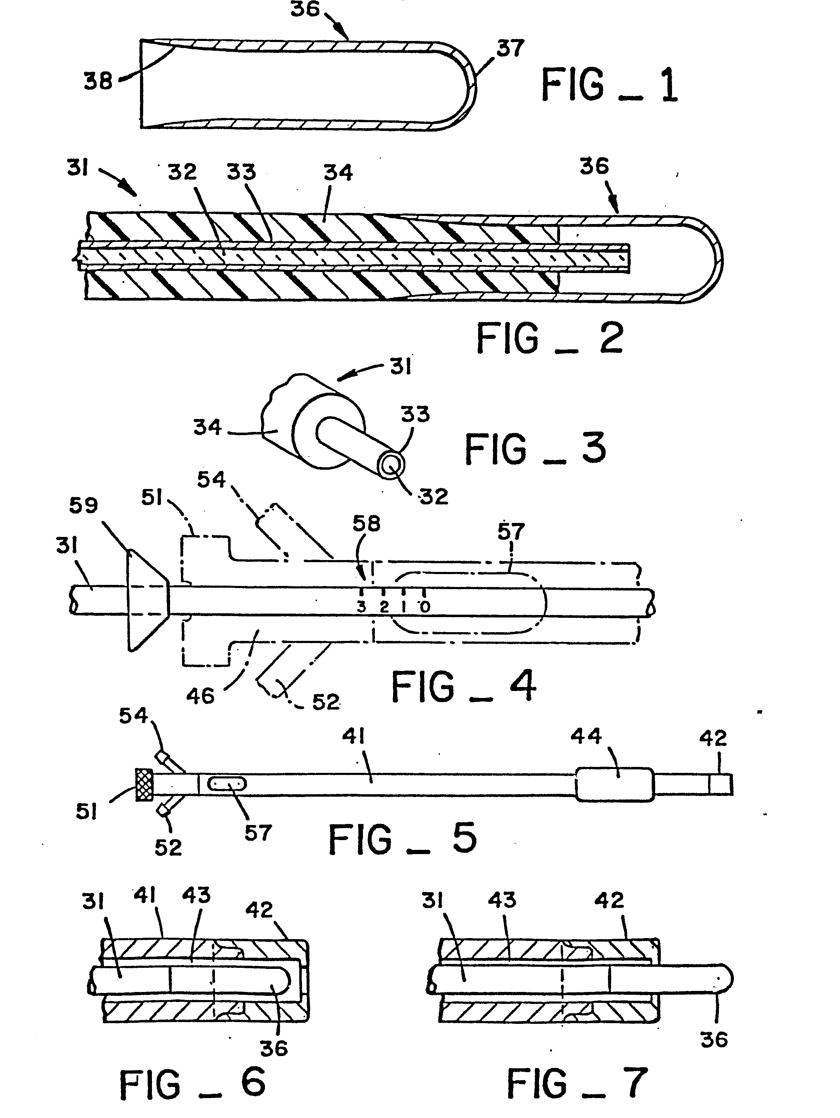

With regard to FIGURES 1-3, one component of the present invention is a fiberoptic bundle or core assembly 31. The assembly 31 includes a fiberoptic core 32 which is provided with a silicone coating 33 thereabout and a flexible exterior cladding 34. The core is approximately 200, 400 or 600 microns or the like in diameter, and the silicone coating limits light leakage from the fiber. The exterior cladding is formed by tetrafluoroethylene or the like to contain and protect the core while providing flexibility to the assembly.

A salient feature of the present invention is the provision of a cautery cap 36 which is adapted to be secured to the distal end of the fiberoptic assembly 31. The cap 36 comprises a cylindrical member having a smoothly rounded, convex, closed distal end 37. The proximal end of the cap is open, and is provided with a interior annular surface 38 which tapers outwardly adjacent to the open end. The distal end of the fiberoptic assembly 31 is provided with a complementary taper to receive and secure the cap.

The cautery cap is joined to the fiberoptic assembly 31 by means of a heat-shrink process, although adhesives or other methods may also be used. The cap is heated to expand the bore thereof, and quickly pressed onto the end of the assembly 31. The cap melts the surface of the tetrafluoroethylene cladding, which then cools and adheres to the interior surface of the cap. At the same time, cooling causes the cap to shrink and tightly grip the cladding.

The cautery cap is designed to absorb the directed energy of a laser which is conducted through the core 32. The outside diameter of the cap is selected within a range of approximately 0.5-1.0 mm or larger, and the wall thickness is approximately 0.002 inch. Thus the mass of the cap is quite low, not only so that laser pulses will cause virtually instantaneous heating of the cap, but also so that little heat will be retained after the laser illumination ceases.

Another component of the instrument of the present invention is a guide sleeve 41, as shown in FIGURES 4 and 5. The guide sleeve comprises a hollow, tubular, flexible member formed of an inner hollow core of inert flexible plastic, a concentric layer of circumferentially wound wire braid, and an outer concentric layer of inert, non-thrombogenic flexible plastic. The bore of the guide sleeve is dimensioned to receive the fiberoptic assembly 31 therein with a minimum gap 43 therebetween of approximately 0.05 mm. Joined to the guide sleeve at the distal end thereof is a terminal hub 42. The terminal hub comprises an annular stainless steel element having a bore substantially the same as the bore of the guide sleeve, and a blunt end face through which the cautery cap may be extended, as shown in FIGURE 7. The hub 42 protects the distal end of the guide sleeve from the heat generated by the cautery cap during laser illumination. When retracted, as shown in FIGURE 6, the cap 36 is nested in protected fashion within the terminal hub. The gap 43 between the assembly 31 and bore of the guide sleeve permits free translation of the assembly within the sleeve, and also permits fluid flow at low flow rates from the proximal end of the sleeve to the terminal hub.

Closely adjacent to the terminal hub is an externally mounted inflatable balloon 44 joined to the guide sleeve 41. As is true of commercially available balloon catheters, the balloon 44 is inflatable

0178464

through a channel in the guide sleeve to expand and immobilize the sleeve in the blood vessel. The expanded balloon also blocks fluid flow within the vessel. Furthermore, the balloon centers the distal end and the terminal hub 42 within the blood vessel, so that there is minimal opportunity for the cautery cap to impinge on the interior surface of the blood vessel.

Secured to the proximal end of the guide sleeve 41 is a proximal hub 46, as shown in FIGURES 4, 5, and 8. The proximal hub 46 comprises a tubular stainless steel housing having a port 47 disposed coaxially with the bore of the guide sleeve and adapted to receive the fiberoptic assembly 31 therethrough in slidable fashion. The port 47 includes an O-ring seal 48 and a threaded bore 49 which receives a nut 51. The fiberoptic assembly also extends through the nut 51, which may be threaded into the port to selectively lock the assembly 31 in place as it extends therethrough. The nut 51 selectively compresses the O-ring seal 48 to seal with the fiberoptic core and prevent the backflow of blood or other fluid from the site of revascularization. The distal end of the hub 46 is provided with a luer connection which engages a ferrule 50 joined to the proximal end of the guide sleeve 41.

The proximal hub 46 also includes a fluid port 52 extending obliquely outwardly therefrom. The port includes a fluid channel which is disposed in registration and fluid communication with a fluid channel 53 within the guide sleeve wall. The fluid channel 53 extends to the exterior balloon 44, and the port 52 provides a connector means to apply a source of pressure, such as a syringe or other hydraulic

13

0178464

source, selectively to inflate (and deflate) the balloon 44.

Also extending obliquely from the hub 46 is another fluid port 54 in opposed relationship to the port 52. The port 54 provides a fluid channel connecting directly to the axial bore 56 extending through the hub 46 and the nut 51 which receives the fiberoptic assembly therethrough. The port 54 permits the injection of cooling or irrigating fluid through the guide sleeve to the distal end to cool the cautery cap during and after each revascularization procedure, and also to flush and suction tissue debris from the vessel. A syringe or other similar device may be connected to the port 54 for this purpose, whether or not the fiberoptic bundle is disposed within the guide sleeve.

The guide sleeve 41 is also provided with a small transparent window 57 secured in the sidewall thereof adjacent to the proximal hub 46. The fiberoptic bundle 31 is likewise provided with numerical indicia 58 which decrement as they progress distally. The indicia provide a reliable reading of the advance of the cautery cap outwardly of the terminal hub, as shown in FIGURE 7. In this regard, it should be noted that the proximal end of the fiberoptic bundle is provided with a stop member 59 secured thereto at a point determined to limit the extension of the cautery cap from the terminal hub to a safe amount.

It should be noted that the guide sleeve assembly is inserted into a blood vessel, and in turn it provides a passageway for the accurate placement of the fiberoptic assembly and the cautery cap. It is also designed, in conjunction with the exterior balloon, to maintain the cautery cap in coaxial

14                    **0178464**

alignment with the longitudinal axis of the blood vessel, ensuring that angular divergence and perforation of the blood vessel does not occur. It further provides a reference scale and a stop to accurately advance the cautery cap from the terminal hub without the risk of blood vessel perforation. The guide sleeve also acts as a conduit for injection and removal of coolant or irrigation fluid, or for contrast medium for fluoroscopic examination. The guide sleeve may also be used to introduce an endoscopic fiberoptic instrument to the site of the atherosclerotic plaque, so that the nature of the plaque and the progress of the revascularization procedure may be assessed.

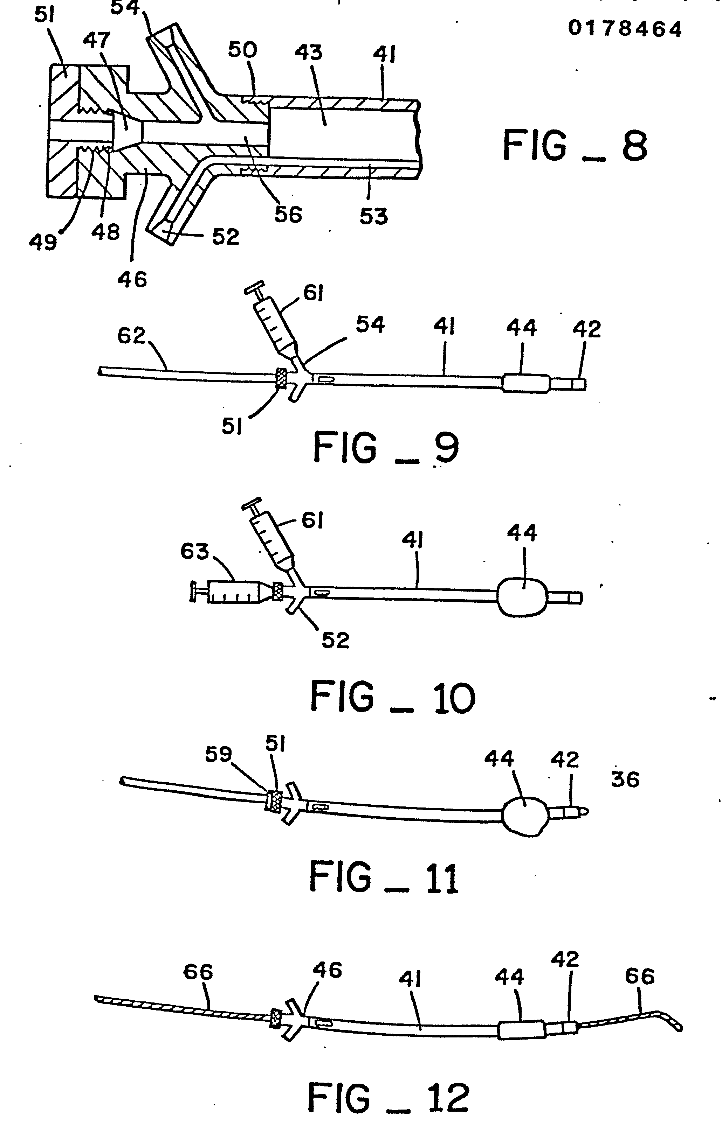

The versatility of the instrument of the present invention may be appreciated with reference to FIGURES 9-12. With the fiberoptic assembly removed, the device as in FIGURE 9 may be configured to introduce flushing solution from a syringe 61 to the outlet at the distal end of the guide sleeve. A reduced pressure suction line 62 may be connected through the nut 51 to the port 47 normally used to receive the fiberoptic assembly. The irrigation solution injection may be alternated with the suction, so that all debris from the revascularization procedure is removed.

With regard to FIGURE 10, the device is shown in a similar configuration, with the flushing fluid being provided by the syringe 61 and the suction provided by another syringe 63. The balloon 44 is depicted in the inflated condition; this inflation may be maintained by a check valve mounted interiorly or exteriorly of the fluid port 52. In FIGURE 11, the fiberoptic assembly is disposed within the guide sleeve, with the stop 59 abutting the nut 51. The cautery cap 36 is thus extended the maximum amount from the terminal hub 42. Furthermore, as depicted in FIGURE 12, the guide sleeve may itself be introduced into a blood vessel which describes a tortuous path. As will be explained in detail in the following, a preformed guide wire 66 is first introduced into the tortuous blood vessel, the wire being smaller in diameter than the bore of the guide sleeve. With the balloon 44 deflated, the proximal end of the guide wire 66 is inserted into the distal end of the guide sleeve assembly. The guide sleeve is then translated along the wire into the blood vessel and to the site of the atherosclerotic plaque.

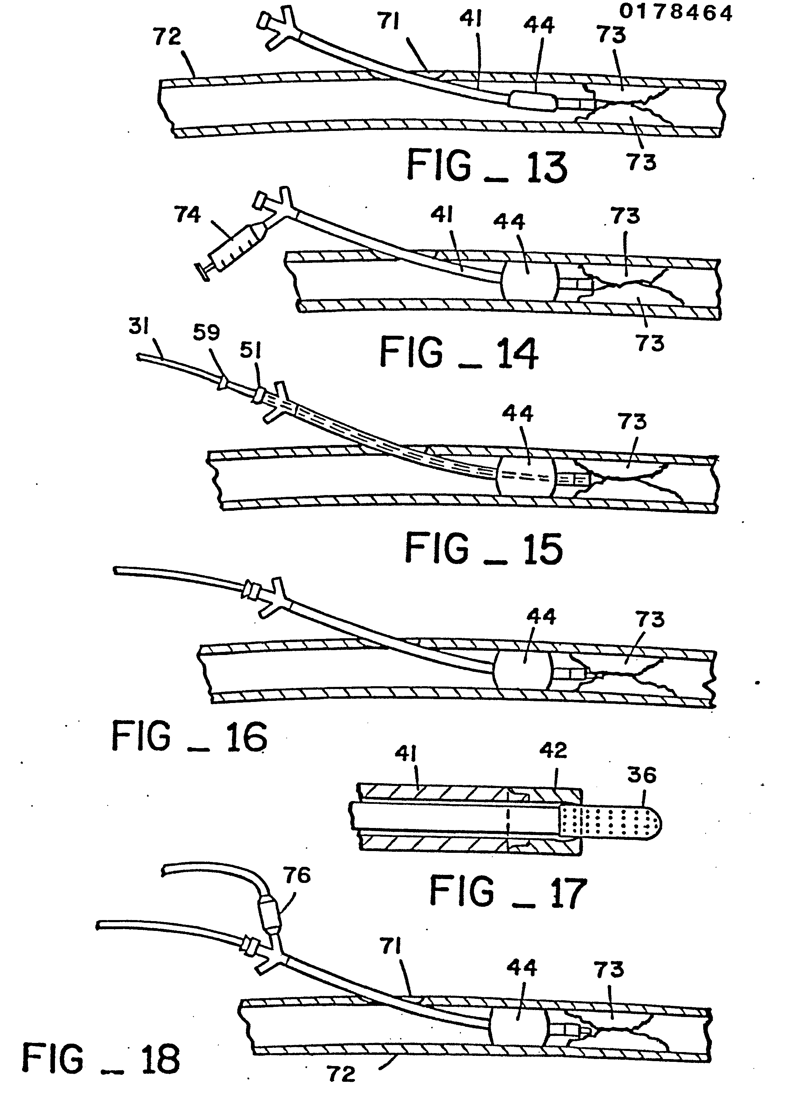

The method of the present invention, which comprises the revascularization procedure accomplished by the instrument described in the foregoing, is depicted somewhat schematically in FIGURE 13 et seq. It is generally characterized as the thermal dissolution of obstructing atherosclerotic plaque in vessels, while the vessel is maintained structurally intact at the site of the obstruction. As shown in FIGURE 13, in blood vessels which present a direct-line approach to the obstruction, an arteriotomy 71 is performed in an artery 72, and the guide sleeve is advanced into the vessel. The guide sleeve may contain an endoscopic fiberoptic member at this time, or it may house the fiberoptic assembly 31. The cap 36 is retracted and the balloon 44 is deflated as the guide sleeve is advanced until the terminal hub 42 abuts the site of the obstructive plaque deposit 73. As shown in FIGURE 14, a syringe 74 is then applied to port 52 of the proximal hub to inflate the balloon 44 and immobilize the guide sleeve assembly in the vessel 72.

The fiberoptic assembly 31 is then advanced through the guide sleeve bore (FIGURE 15), and the cautery cap extends through the terminal hub (FIGURE 17) until the cap contacts the plaque deposit 73 (FIGURE 16). A coolant fluid reservoir 76 under low pressure is connected to the port 54 (FIGURE 18) to pump a steady flow of coolant fluid to the terminal hub and past the cautery cap.

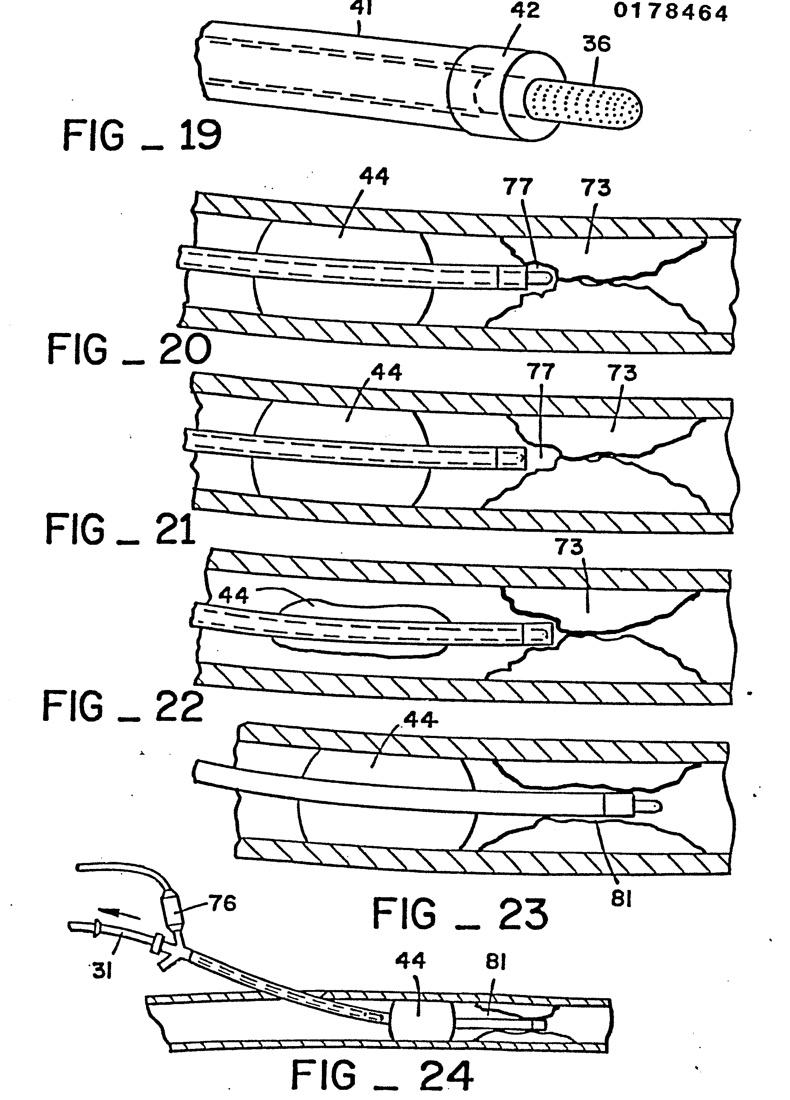

Pulsed mode laser energy is then directed along the fiberoptic assembly 31 to the cautery cap 36, which heats to at least 600°C within one second. As shown in FIGURE 20, this thermal pulse applied to the plaque 73 destroys an annular volume 77 of the plaque which was in contact with the cap 36. The plaque tissue in contact with the cap undergoes dessication and charring; the loss of tissue water and other volatile materials results in collapse of cellular structures and reopening of the vascular lumen. The cautery cap is then withdrawn into the terminal hub 42, and the balloon 44 is deflated, as shown in FIGURE 22. The guide sleeve may then be advanced so that the terminal hub occupies the newly opened space 77, and the balloon is reinflated. The cautery cap is then advanced from the terminal hub once more, laser energy is applied to destroy the newly adjacent plaque, and the process is reiterated until the newly opened serial spaces 77 define a new channel 81. The lumen has thus been recanalized.

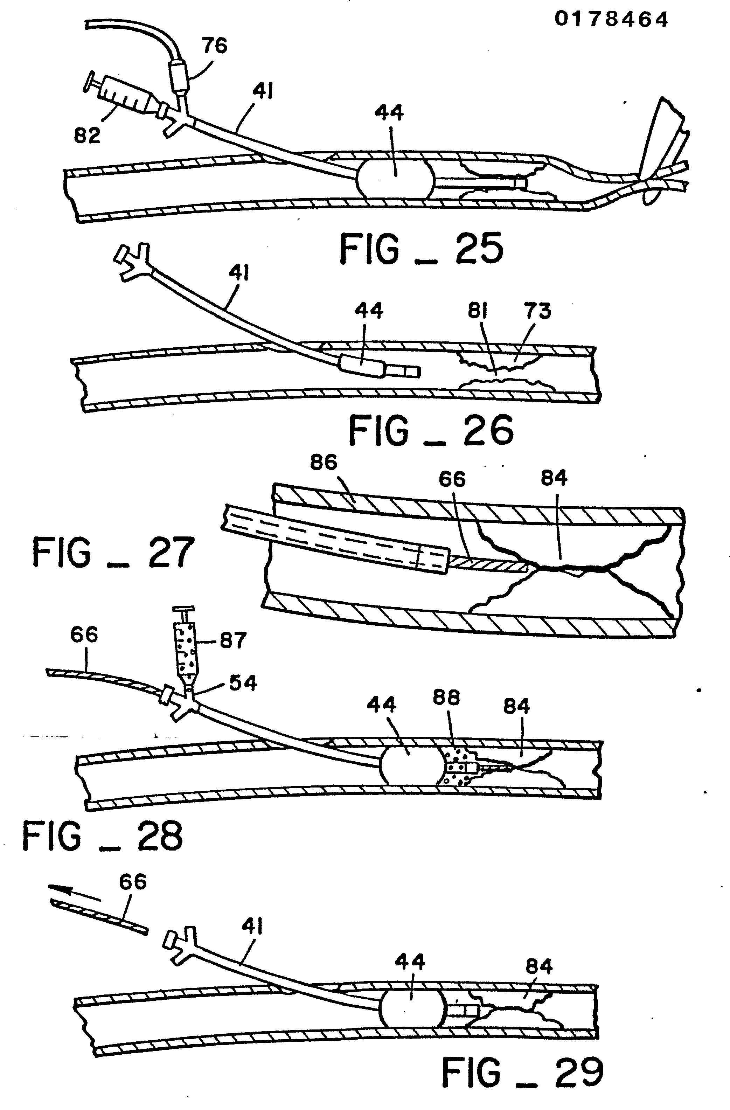

With the balloon 44 still inflated, the fiberoptic assembly 31 is removed from the guide sleeve. A flushing solution is then pumped through the guide sleeve to the plaque site, and suctioned out by syringe 82. The site may then be checked visually by means of an endoscopic fiberoptic core inserted into and through the guide sleeve. The balloon is then deflated, and the guide sleeve is withdrawn from the arteriotomy (FIGURE 26).

As described with regard to FIGURE 12, a wire guide 66 may be employed to direct the guide sleeve through a tortuous blood vessel 86 to an obstructive plaque deposit 84, as shown also in FIGURE 27. The wire guide is first introduced through an arteriotomy, and, by virtue of its flexibility it is translated to the plaque site. The guide sleeve assembly, with the assembly 31 removed, is then secured about the wire guide 66, and advanced through the vessel 86 to the plaque. When the terminal hub abuts the plaque, the wire is withdrawn (FIGURE 29) and the fiberoptic assembly introduced through the sleeve. The balloon 44 is inflated through port 52, and a syringe 87 is applied to port 54 to inject a fluoroscopic contrast medium 88 into the vessel adjacent to the plaque deposit (FIGURE 28). A fluoroscopic position check is then performed, and if the position is suitable, the revascularization procedures are performed as described in the foregoing.

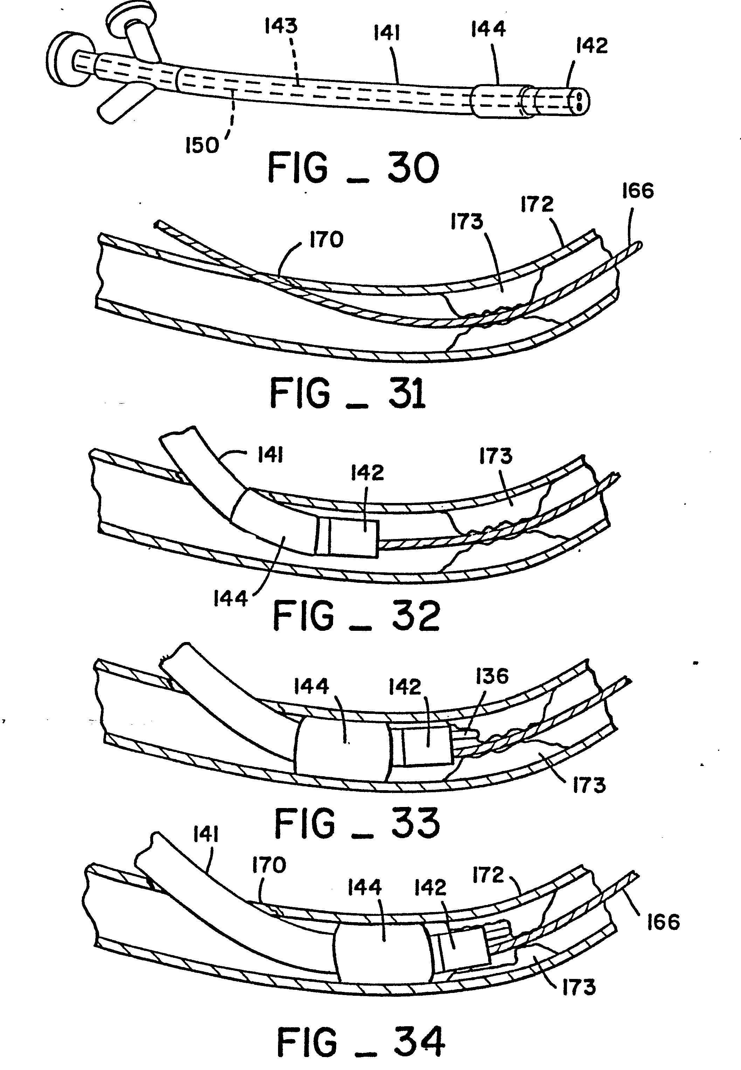

With regard to FIGURE 30, a further embodiment of the present invention is designed for continuous or rapidly reiterated use. It includes a flexible guide sleeve 141 similar in outward appearance to the guide sleeve 41 described above, but provided with a longitudinal bore 143 for the fiberoptic core assembly, and another longitudinal bore 150 which is adapted to receive a flexible guide wire 166. In a vessel 172 with plaque deposits 173 formed therein but not totally obstructive, the guide wire 166 is first inserted through an arteriotomy 170 and advanced through the vessel, as shown in FIGURE 31. The sleeve 141 is then advanced about the guide wire, with the fiberoptic assembly (such as the assembly 31) disposed within the sleeve, as depicted in FIGURE 32.

At any point along the vessel where plaque is encountered, the cautery cap 136 may be advanced from the terminal hub 142 to destroy the plaque deposits by heating. The balloon 144 is first inflated to stabilize the position of the instrument, and the cap is then extended and actuated to heat and ablate the plaque deposits. The plaque deposit is thus diminished, as shown in FIGURE 33, and the debris are removed by flush/suction as described in the foregoing.

It is significant to note that the exit ports for the cap and the guide wire are positioned eccentrically in the terminal hub 144. This placement permits rotation of the sleeve about the guide wire, so that the cautery cap may sweep through a large annular volume center about the guide wire axis. The cap may be actuated reiteratively to destroy all the plaque within the swept annular space, as shown in FIGURE 34, thus opening a substantial channel through the plaque deposit 173.

It may be appreciated from the preceding description that the present invention provides an instrument and a method for restoring blood circulation to blocked blood vessels by recanalizating the lumen which is blocked by plaque deposits. The invention is designed to maximize the safety of the patient by using a captive laser beam rather than a free beam propagated within the body. The design further eliminates damage to surrounding tissue by accurately controlling the position of the cautery cap, and by providing fluid cooling of the cap and fluid flushing of the atherosclerotic plaque debris after recanalization.

WHAT IS CLAIMED IS:

1. A device for applying heat to a body site, including; an elongated light transmitting conduit having a proximal end and a distal end, a controlled light source adapted to be directed through said conduit to said distal end, and a heat generating element operatively associated with said distal end to receive the light energy from said controlled light source and to convert said light energy to heat energy.

2. The device of claim 1, wherein said light conduit comprises a fiberoptic assembly.

3. A device of claim 1, wherein said controlled light source is a laser device.

4. The device of claim 1, wherein said heat generating element comprises a member joined to the distal end of said light transmitting conduit.

5. The device of claim 4, wherein said member comprises an opaque cup secured to said distal end of said light transmitting conduit.

6. A method for removing at least a portion of material causing a constriction or obstruction in a lumen, comprising the steps of: providing a device having a heat generating element mounted on the distal end of a light transmitting conduit; positioning the distal end of the conduit within the lumen and in contact with the material; transmitting sufficient light energy through the conduit to heat the heat generating element and cause thermal destruction of the material contacting the element.

7. The method of claim 6, further including the step of cooling the element with a fluid flow thereby to limit damage to surrounding healthy tissue.

8. The method of claim 6, wherein said light energy comprises the pulsed output of a laser light source.

9. The method of claim 6, wherein said heat generating element is provided with a low thermal mass to undergo extremely fast heating and cooling cycles.

10. A device for luminal recanalization of atherosclerotic blood vessels, including; a flexible guide sleeve having a bore therethrough and adapted to be introduced into a blood vessel and advanced to the atherosclerotic plaque; a light conduit having a proximal end adapted to be connected to a controlled light source and a distal end including a heat generating element, said conduit being dimensioned to be received within the bore of said sleeve in freely translating fashion, said guide sleeve including a distal end, and means for advancing said conduit so that said heat generating element extends from said distal end of said guide sleeve a controlled and limited amount.

11. The device of claim 10, wherein said light conduit includes a fiberoptic core.

12. The device of claim 10, wherein said bore of said guide sleeve is larger in diameter than said light conduit therein to define an annular space therebetween extending the length of said guide sleeve, and means for pumping fluid through said annular space to said distal end of said guide sleeve.

13. The device of claim 12, further including an inflatable balloon secured to the exterior of said guide sleeve adjacent to said distal end thereof.

14. The device of claim 13, further including a fluid channel extending from the proximal end of said guide sleeve to said balloon for selective inflation and deflation thereof.

15. The device of claim 14, further including a proximal hub joined to the proximal end of said guide sleeve.

16. The device of claim 15, wherein said proximal hub includes a closed housing having a first port disposed generally coaxially with said guide sleeve bore and adapted to receive said light conduit therethrough.

17. The device of claim 15, wherein said proximal hub also includes means for selectively securing said light conduit at any longitudinal location therealong.

0178464

18. The device of claim 16, further including a second port in said proximal hub connected to said annular space for pumping a fluid into said annular space.

19. The device of claim 18, further including a third port in said proximal hub connected to said fluid channel to facilitate inflation and deflation of said balloon.

20. The device of claim 10, wherein said heat generating element comprises a cautery cap secured to the distal end of said light conduit.

21. The device of claim 20, wherein said cautery cap comprises a generally cylindrical member of low thermal mass, said member having a closed, convex distal end, and an open proximal end adapted to be secured about said light conduit.

22. The device of claim 10, wherein said guide sleeve includes a second bore extending therethrough and adapted to receive a guide wire therethrough while said light conduit is disposed within said first mentioned bore of said guide sleeve.

23. The device of claim 22, wherein said heat generating element is disposed eccentrically with respect to said guide wire, said device being rotatable to cause said heat generating element to sweep through a large annular volume centered about said guide wire.

FIG _ 1

FIG _ 2

FIG _ 3

FIG _ 4

FIG _ 5

FIG _ 6

FIG _ 7

0178464

FIG _ 8

FIG _ 9

FIG _ 10

FIG _ 11

FIG _ 12

0178464

FIG _ 13

FIG _ 14

FIG _ 15

FIG _ 16

FIG _ 17

FIG _ 18

0178464

**FIG _ 19**

**FIG _ 20**

**FIG _ 21**

**FIG _ 22**

**FIG _ 23**

**FIG _ 24**

FIG _ 25

FIG _ 26

FIG _ 27

FIG _ 28

FIG _ 29

0178464

FIG _ 30

FIG _ 31

FIG _ 32

FIG _ 33

FIG _ 34